# FASCICULE DE BREVET EUROPEEN

(11) **EP 0 957 162 B1**
(45) Date de publication et mention de la délivrance du brevet: **10.08.2005**
(21) Numéro de dépôt: 99113896.7
(22) Date de dépôt: 02.04.1993
(51) Int. Cl.: C12N 7/00, C12N 5/08, C12N 5/10, C12N 15/38, C12N 15/85

(54) **Matériel biologique viral issu d'une lignée cellulaire infectée par une souche virale associée à la sclérose en plaques**
Aus einer mit einem Stamm von Multiple-Sklerosis-Virus infizierten Zellinie hergestelltes biologisches Virusmaterial
Biological viral material obtained from a multiple sclerosis-associated virus strain infected cell line

(30) Priorité: 03.04.1992 FR 9204322; 03.11.1992 FR 9213443
(43) Date de publication de la demande: 17.11.1999
(62) Demande divisionnaire de: 93908989.2
(73) Titulaire: BIO MERIEUX, 69280 Marcy l'Etoile (FR)
(72) Inventeur: Perron, Hervé, 69005 Lyon (FR); Seigneurin, Jean-Marie, 39190 Bernin (FR)
(74) Mandataire: Guerre, Dominique

(56) Documents cités:
- H. PERRON ET AL.: "LEPTOMENINGEAL CELL LINE FROM MULTIPLE SCLEROSIS WITH REVERSE TRANSCRIPTASE ACTIVITY AND VIRAL PARTICLES." RESEARCH IN VIROLOGY, vol. 140, no. 6, 1989, pages 551-561, XP002115003 PARIS, FR
- H. PERRON ET AL.: "ISOLATION OF RETROVIRUS FROM PATIENTS WITH MULTIPLE SCLEROSIS." THE LANCET, vol. 337, no. 8745, 6 avril 1991 (1991-04-06), pages 862-863, XP000673661 LONDON, GB
- H. PERRON ET AL.: "ANTIBODY TO REVERSE TRANSCRIPTASE OF HUMAN RETROVIRUSES IN MULTIPLE SCLEROSIS." ACTA NEUROLOGICA SCANDINAVICA, vol. 84, no. 6, décembre 1991 (1991-12), pages 507-513, XP002115004 COPENHAGEN, DK
- J.D. MOSCA ET AL.: "ACTIVATION OF HUMAN IMMUNODEFICIENCY VIRUS BY HERPESVIRUS INFECTION: IDENTIFICATION OF A REGION WITHIN THE LONG TERMINAL REPEAT THAT RESPONDS TO A TRANS-ACTING FACTOR ENCODED BY HERPES SIMPLEX VIRUS 1." PROCEEDINGS OF THE NATIONAL ACADEMY OF SCIENCES OF USA, vol. 84, no. 21, novembre 1987 (1987-11), pages 7408-7412, XP002115005 WASHINGTON US
- H. PERRON ET AL.: "HERPES SIMPLEX VIRUS ICPO AND ICP4 IMMEDIATE EARLY PROTEINS STRONGLY ENHANCE EXPRESSION OF A RETROVIRUS HARBOURED BY A LEPTOMENINGEAL CELL LINE FROM A PATIENT WITH MULTIPLE SCLEROSIS." THE JOURNAL OF GENERAL VIRILOGY, vol. 74, no. 1, janvier 1993 (1993-01), pages 65-72, XP002115006 COLCHESTER, GB
- PERRON H. ET AL.: "Cell cultures and associated retrovirus in multiple sclerosis" ACTA NEUROLOGICA SCANDINAVICA, vol. 169, - 1997 pages 22-31,
- PERRON H. ET AL.: "In vitro transmission and antigenicity of a retrovirus isolated from a multiple sclerosis patient" RES. VIROL., vol. 143, - octobre 1992 (1992-10) pages 337-350,

## Description

La présente invention a pour objet une souche virale humaine, à l'état isolé, associée à la sclérose en plaques, dénommée POL-2. Cette souche est hébergée par une lignée cellulaire PLI-2 qui fait l'objet de la demande de brevet européen principal 93 908 989.2 (EP-587 873), ainsi que son procédé d'obtention.

La sclérose en plaques (SEP) est une maladie démyélinisante du système nerveux central (SNC) que l'on suspecte depuis plusieurs années être associée à un virus, bien que l'agent causal n'ait pas encore été déterminé avec certitude.

De nombreux travaux ont étayé cette hypothèse d'une étiologie virale de la maladie, mais aucun des virus connus testés ne s'est avéré être l'agent causal recherché.

Par la suite, l'observation chez des patients atteints de sclérose en plaques de phénomènes assimilables à une réaction d'auto-immunité a conduit à une hypothèse étiologique auto-immune "essentielle" (Lisak R.P., Zweiman B. New Engl. J. Med. 1977; 297, 850-853, et Lassmann H. et Wisniewski H.M. Arch. Neurol. 1979; 36, 490-497). Cependant, cette auto-immunité dirigée contre certains composants du système nerveux central s'est révélée peu spécifique de la SEP et fréquente dans des inflammations du SNC, associées ou non à une infection, comme cela a été montré par Hirayama M. et al. (Neurology 1986 ; 36, 276-8) Kenneth G. Warren et al. (Annals of Neurology 1986 ; 20, 20-25), Suzumura A. et al. (Journal of Neuroimmunology 1986 ; 11, 137-47) et Tourtelotte W. et al. (Journal of Neurochemistry 1986 ; 46, 1086-93). De plus, comme l'a fait remarquer E.J. Field (The Lancet 1989 ; 1, 1272), aucune des thérapeutiques immunosuppressives n'a permis d'obtenir des résultats décisifs contre la SEP.

Une hypothèse a été émise selon laquelle un rétrovirus serait à l'origine de la maladie. La découverte par A. Gessain et al. (J. Infect. Disease 1988 ; 1226-1234), de syndromes neurologiques associés au virus HTLV-1, connu à l'origine comme agent de leucémies T de l'adulte, a conduit de nombreux auteurs tels que H. Koprowski et al. (Nature 1985 ; 318, 154), M. Ohta et al. (J. Immunol. 1986 ; 137, 3440), E.P. Reddy et al. (Science 1989 ; 243, 529), S.J. Greenberg et al. (Proc. Natl. Acad. Sci. USA 1989 ; 86, 2878), J.H. Richardson et al. (Science 1989 ; 246, 821), S.L. Hauser et al. (Nature 1986 ; 322, 176) et A. Karpas et al. (Nature 1986 ; 322, 177), à rechercher une implication de ce rétrovirus humain dans la SEP, cependant sans succès ou avec des résultats évoquant des réactions croisées.

Il existe par ailleurs un modèle animal très proche de la SEP, induit par un rétrovirus : le virus MAEDI-VISNA du mouton. Il est connu que l'infection naturelle par ce virus provoque une maladie ovine proche de la SEP, comme le rapportent Johnson R.T. (Rev. Infect. Dis. 1985 ; 7, 66-67), Narayan O. et Cork L.C. (Rev. Infect. Dis. 1985 ; 7, 89-98) et Nathanson N. et al. (Rev. Infect. Dis. 1985 ; 7, 75-82). L'infection expérimentale de moutons par inoculation intra-ventriculaire de souches neurovirulentes du virus VISNA a permis d'établir la responsabilité de ce virus dans la génèse de cette infection démyélinisante du mouton. Comme l'expliquent Nathanson N. et al. (Rev. Infect. Dis. 1985 ; 7, 75-82), Hoffman P.M. et Panitch H.S. ("Handbook of Clinical Neurology, 12 ; Viral diseases" R.R. Mc Kendall, ed., Elsevier science Publishing, Amsterdam, 1989, 453-466) et A. Haase (Nature 1986 ; 322, 130-136), elle diffère quelque peu de l'infection naturelle, mais reste néanmoins proche de la SEP. Il est par ailleurs intéressant de noter que dans l'ensemble des travaux effectués sur ce sujet par les auteurs précités, le virus Visna est retrouvé dans les cellules de plexus choroïdes du cerveau des moutons infectés qui constituent un site de latence et de réplication occasionnelle du provirus Visna ; la localisation de ces cellules à l'interface sang/liquide céphalo-rachidien expliquant certainement ce phénomène.

Tous ces résultats plaident en faveur du rôle dans la SEP d'un rétrovirus inconnu.

Récemment, les travaux de H. PERRON et al. (Res. Virol. 1989 ; 140, 551-561, et "Current concepts in multiple sclerosis" Wiethölter et al., eds. Amsterdam, Elsevier, 1991, p. 111-116 et The Lancet 1991 ; 337, 862-863) ont permis, à partir d'une ponction lombaire de liquide céphalo-rachidien d'un patient souffrant de SEP d'isoler une lignée de cellules non lymphoïdes et de mettre en évidence la présence d'un virus, présentant les caractéristiques d'un rétrovirus et montrant en particulier une activité transcriptase inverse, dans le surnageant de culture des cellules de cette lignée. L'étude au microscope électronique des cellules de cette lignée a permis de mettre en évidence des particules virales d'un diamètre compris environ entre 110 et 140 nm, la taille des particules variant selon qu'il s'agit de particules matures ou immatures. Par ailleurs, une étude sérologique, selon la technique ELISA, utilisant un extrait cellulaire des cellules infectées de cette lignée, a permis de montrer, avec 40 sérums de patients parmi lesquels 20 sont atteints de SEP (SEP certaine) et 20 sont des malades présumés (SEP probable), 60 % de résultats positifs. Une étude comparative avec 40 sérums de patients atteints d'autres maladies neurologiques que la SEP n'a donné que 5 % de résultats positifs. Cette lignée, que les auteurs ont dénommée LM7, est clonale et non-immortelle et son étude immunocytochimique et ultrastructurale a permis de caractériser son origine leptoméningée.

Ce virus s'est avéré cependant très difficile à étudier, du fait que d'une part il s'exprime très faiblement , in vitro, dans la lignée cellulaire primaire d'origine leptoméningée, et que d'autre part cette lignée cellulaire dégénère assez rapidement après une trentaine de passages par extinction de son pouvoir mitotique, de sorte qu'elle ne permet plus l'expression virale.

Aussi, les auteurs ont envisagé une nouvelle approche (H. Perron et al., The Lancet, vol. 337, 862-863, (1991)), qui consiste à prélever un échantillon sanguin chez un patient atteint de SEP, à effectuer une culture de monocytes et à recueillir le surnageant pour vérifier l'expression d'une activité transcriptase inverse, soit directement dans le culot d'ultracentrifugation, soit après sédimentation à l'équilibre sur gradient de saccharose. Il a ainsi été montré un pic d'activité transcriptase inverse dans le surnageant chez les patients atteints de SEP et que cette activité est retrouvée dans la fraction de densité 1,17 g/ml environ. Une étude au microscope électronique des cellules infectées a permis de mettre en évidence des particules semblables à des rétrovirus de 100 à 120 nm qui sont retrouvées dans les culots d'ultracentrifugation de surnageants de cultures exprimant une activité transcriptase inverse élevée. Les culots de centrifugation contenant des débris cellulaires et potentiellement des particules virales ont ensuite été cultivés sur des cellules du sang de cordon, pour la mise en évidence d'une expression virale. Cependant, comme expliqué par les auteurs, un effet cytopathique a été observé dans les cellules de sang de cordon infectées, mais n'est plus détectable six semaines après l'inoculation, de sorte que ce mode de culture n'est pas satisfaisant pour une étude approfondie des caractéristiques de ce virus.

Il était donc indispensable de disposer d'un procédé pour la culture in vitro de cellules infectées par ce virus.

Une hypothèse a été émise et vérifiée selon laquelle des cellules permissives de plexus choroides humains pourraient être des cellules permissives au virus rétrouvé chez des patients atteints de SEP. Sur la base de cette découverte, un procédé de culture in vitro de cellules infectées par un virus associé à la SEP a été développé.

Le procédé consiste à effectuer une culture de cellules de plexus choroïdes, obtenues après explantation post mortem de plexus choroïdes humains, dans un milieu approprié comprenant, des acides aminés, des facteurs vitaminiques, des sels inorganiques et du glucose, respectivement à des concentrations pondérales totales comprises entre 400 et 2250 mg/l, 3,5 et 130 mg/l, 9100 et 13000 mg/l, et 1000 et 6000 mg/l, additionné avantageusement d'un facteur de croissance, tel qu'ECGF ("Endothelial Cell Growth Factor"), pour favoriser la croissance des cellules et d'au moins un antibiotique, puis à mettre en contact les cellules de plexus choroïdes ainsi cultivées, dans leur milieu de culture, avec des cellules primaires ou dérivées, infectées ou un surnageant de culture contenant le virus dans des conditions déterminées permettant la propagation du virus des cellules infectées aux cellules cultivées, sa replication et son expression.

Par cellules dérivées des cellules primaires, on entend toute culture obtenue directement ou indirectement à partir desdites cellules primaires, par exemple par conservation à basse de température ou maintien de la viabilité desdites cellules. Il peut s'agir par exemple de cellules de référence déposées dans une collection.

Cependant, la propagation du virus à partir des quelques cellules infectées, bien qu'existante, reste relativement restreinte au cours des passages successifs et necessite de nombreux passages pour obtenir un niveau d'expression suffisant. La durée de vie en culture de ces cellules étant limitée, ce n'est souvent qu'aux derniers passages que l'expression devient quantifiable, ce qui limite considérablement l'intérêt dudit procédé.

Les travaux des présents inventeurs les ont conduit à la mise en évidence tout-à-fait surprenante de la production d'interféron beta par les cellules de plexus choroïdes en réponse à une agression virale. Or, seuls les fibroblastes, cellules épithéliales et macrophages sont connus à ce jour pour produire l'interféron beta (Interféron : Principles and Medical Applications, the University of Texas Medical Branch at GALVESTON, Department of Microbiology, GALVESTON, TX, 1992). Les effets des interférons sont bien connus, et notamment ils induisent un état réfractaire des cellules à la synthèse et à la replication de matériel viral, inhibant ainsi la propagation et la production des virus en culture. Il devenait alors fortement probable que la production d'interféron beta par les cellules de plexus choroïdes soit un facteur limitant déterminant dans le procédé de culture in vitro de cellules infectées par un virus présent chez les individus atteints de sclérose en plaques.

Sur la base de cette découverte inattendue, les présents inventeurs ont mis au point un nouveau milieu de culture utilisable dans un procédé de culture in vitro de cellules infectées par un virus retrouvé chez des patients atteints de SEP.

Un milieu approprié pour la culture in vitro de cellules infectées par un virus présent chez les individus atteints de sclérose en plaques comprend, outre des acides aminés, des facteurs vitaminiques, des sels inorganiques et du glucose, respectivement à des concentrations pondérales totales comprises entre 400 et 2250 mg/l, 3,5 et 130 mg/l, 9100 et 13000 mg/l, et 1000 et 6000 mg/l, et au moins un anticorps anti-interféron beta.

Plus particulièrement, le milieu comprend au moins les constituants suivants:
* un ou plusieurs acides aminés choisis parmi les composés suivants :
   arginine: 100 à 500 mg/l, de préférence 100 à 300 mg/l cystéine et /ou cystine: 25 à 300 mg/l, de préférence
   cystine: 25 à 100mg/l
   glutamine: 200 à 1000 mg/l, de préférence 200 à 500 mg/l
   histidine : 5 à 50 mg/l, de préférence 5 à 20 mg/l
   isoleucine : 20 à 100 mg/l, de préférence 20 à 60 mg/l
   leucine : 20 à 100 mg/l, de préférence 20 à 60 mg/l
   lysine : 20 à 100 mg/l, de préférence 20 à 80 mg/l
   methionine : 5 à 50 mg/l, de préférence 5 à 30 mg/l
   phénylalanine : 10 à 70 mg/l, de préférence 10 à 50 mg/l
   thréonine : 15 à 100 mg/l, de préférence 15 à 60 mg/l
   tryptophane : 2 à 30 mg/l, de préférence 2 à 25 mg/l
   tyrosine : 10 à 70 mg/l, de préférence 10 à 50 mg/l
   valine : 10 à 80 mg/l, de préférence 10 à 60 mg/l
* un ou plusieurs facteurs vitaminiques, choisis parmi les composés suivants :
   panthothénate : 0,15 à 5 mg/l, de préférence sel de calcium 0,15 à 2 mg/l
   choline : 0,5 à 10 mg/l, de préférence sel de chlorure 0,5 à 5 mg/l
   acide folique : 0,5 à 10 mg/l, de préférence 0,5 à 5 mg/l
   inositol : 1 à 70 mg/l, de préférence 1 à 50 mg/l
   nicotinamide et/ou niacinamide : 0,5 à 10 mg/l, de préférence nicotinamide 0,5 à 5 mg/l
   pyrridoxine et/ou pyrridoxal : 0,5 à 10 mg/l, de préférence pyrridoxine/HCl 0,5 à 5 mg/l
   riboflavine : 0,05 à 1 mg/l, de préférence 0,05 à 0,5 mg/l
   thiamine : 0,5 à 10 mg/l, de préférence 0,5 à 5 mg/l
* un ou plusieurs sels inorganiques, choisis parmi les composés suivants :
   sels de calcium : 100 à 200 mg/l, de préférence CaCl2 anhydre
   chlorure de potassium : 350 à 450 mg/l
   sels de magnésium : 40 à 60 mg/l, de préférence MgSO₄ anhydre
   chlorure de sodium : 6 000 à 8 000 mg/l
   sels de HCO₃ : 2 000 à 3 000 mg/l, de préférence NaHCO₃
   sels de HPO₄ : 600 à 1 000 mg/l, de préférence Na₂HPO₄ anhydre
* du glucose : 1 000 à 6 000 mg/l, de préférence D-glucose
* anticorps anti-interféron beta : environ 10 U/ml

Avantageusement, les acides aminés sont choisis parmi ceux de la série naturelle L.

Le milieu peut également comprendre au moins un antibiotique, de préférence un mélange de pénicilline et de streptomycine, et si souhaité de la clindamycine pour empêcher les contaminations mycoplasmiques.

Le milieu peut comprendre de plus au moins un facteur de croissance choisi parmi l'ECGF ("Endothelial Cell Growth Factor" dénommé également FGF acide) et le FGF ("Fibroblast Growth Factor") basique dans des proportions variables que l'homme du métier peut déterminer à partir de ses connaissances générales des cultures cellulaires et des produits qu'il a à sa disposition. A titre d'exemple, la concentration du facteur de croissance est comprise entre 1 et 50 µg/l de milieu de culture, en présence d'héparine à une concentration comprise entre 50 et 50 µg/l. Avantageusement, le facteur de croissance choisi est l'ECGF (10 µg/l, en présence d'héparine comme précédemment).

Un procédé d'obtention in vitro d'une culture de cellules de plexus choroïdes humains infectées, prélevées post mortem sur le corps d'un individu ou patient atteint de SEP, comprend les étapes selon lesquelles on cultive lesdites cellules prélevées dans le milieu de culture précité et dans des conditions déterminées pour obtenir une première culture de cellules primaires de plexus choroïdes, puis on cultive en série, c'est-à-dire par passages successifs, dans ledit milieu de culture un prélèvement de ladite culture de cellules primaires ou de sous-culture de cette dernière, pour obtenir une culture de cellules de plexus choroïdes infectées.

Ce procédé a permis d'obtenir une lignée cellulaire infectée de cellules de plexus choroïdes, dénommée PLI-2, déposée à l'ECACC le 22 juillet 1992, sous le numéro 92072201 conformément au Traité de Budapest.

La présente invention a pour objet une souche virale humaine, à l'état isolé, associée à la sclérose en plaques, dénommée POL-2 et hébergée par la lignée cellulaire PLI-2 ci-dessus. Elle a été déposée à l'ECACC le 22 juillet 1992, sous le numéro V92072202 conformément au Traité de Budapest.

Avantageusement, les cellules de la lignée PLI-2 sont transfectées par un gène "immédiat-précoce" d'un virus du genre Herpèsviridae pour accroître l'expression virale dans ces cellules.

Un procédé d'obtention d'une culture cellulaire ou lignée infectée viable, ou continue, comprenant des cellules infectées par au moins une souche virale humaine associée à la SEP, consiste à:
(a) cultiver des cellules humaines infectées par la souche virale, pour obtenir au moins une culture primaire ou dérivée, infectée par ladite souche,
(b) cultiver des cellules humaines permissives, de préférence des cellules de plexus choroïdes humains non infectées obtenues selon le procédé décrit ci-dessus, lesdites cellules permissives étant susceptibles de s'infecter et de repliquer ladite souche virale, pour obtenir au moins une culture permissive,
(c) cocultiver au moins un prélèvement d'une culture primaire infectée et un prélèvement d'une culture permissive, pour obtenir une première culture dérivée infectée par unedite souche virale,
(d) cultiver en série, c'est à dire par repiquages successifs, la première culture dérivée infectée; à cette fin, on répète au cours du temps l'étape consistant à cocultiver, par exemple pendant 5 à 8 jours, un nouveau prélèvement d'une culture permissive non infectée et un prélèvement de la première culture dérivée infectée, ou d'une sous-culture de cette dernière, pour obtenir une nouvelle sous-culture de la même première culture dérivée infectée, constituant une culture virale continue dans des cellules non immortelles.

Par culture dérivée de la culture primaire, on entend toute culture ou sous-culture obtenue directement ou indirectement à partir de ladite culture primaire, par conservation à basse température ou par exemple par maintien de la viabilité de ladite culture. Il peut s'agir par exemple d'une culture de référence, déposée dans une collection.

Au moins l'une quelconque des étapes (a) à (d) est effectuée avec un milieu de culture contenant un anticorps anti-interféron beta.

Avantageusement, la culture primaire infectée est obtenue à partir de cellules humaines infectées par ladite souche virale, résultant du procédé d'obtention in vitro décrit précédemment, par exemple la lignée cellulaire 92072201 de l'ECACC, et/ou à partir de cellules humaines infectées par ladite souche virale, choisies dans le groupe comprenant des cellules leptoméningées, des cellules de plexus choroïdes, des cellules myéloïdes sanguines, notamment des macrophages et monocytes, et des lymphocytes.

Préférentiellement, la culture permissive est obtenue à partir de cellules humaines de plexus choroïdes.

Avantageusement, les cellules hébergeant ladite souche virale continue sont transfectées par au moins un gène "immédiat-précoce" d'un virus du genre Herpèsviridae, pour accroître l'expression virale dans ces cellules.

L'utilisation de sérums ou d'anticorps anti-interféron beta humain a permis d'obtenir une propagation accrue des souches virales présentes dans les cellules explantées à partir des pièces anatomiques, ou introduites par coculture dans des cellules de plexus choroïdes non infectées. Ainsi, l'expression globale du virus dans les cultures des cellules de plexus choroïdes a pu être augmentée et le délai d'obtention d'un signal décelable après mise en culture d'isolats pathologiques a pu être raccourci. Ces effets sont attribuables à la neutralisation par les anticorps de l'interféron beta ou d'une molécule antigéniquement proche, jouant un rôle inhibiteur dans l'expression virale, produit par ces cellules en présence de virus.

Par "anticorps anti-interféron β" on entend toute préparation contenant des anticorps d'origine mono ou polyclonale, purifiés (par exemple par chromatographie d'affinité) ou non, qui reconnaissent des épitopes appartenant à l'interferon β humain ou à toute molécule antigéniquement analogue jouant un rôle inhibiteur dans l'expression virale.

Selon un mode particulier de réalisation du procédé la culture primaire infectée, avant mise en contact avec les cellules permissives cultivées est préalablement traitées par irradiation, par exemple par irradiation par des rayons X.

Selon un mode de réalisation particulier du procédé, on ontient plusieurs cultures primaires infectées par des souches virales ou isolats de SEP recpectivement différents, et pendant l'étape (b), on cocultive des prélèvements desdites cultures primaires ou de sous-cultures de ces dernières, respectivement différents. On obtient ainsi dans la culture cellulaire un mélange de souches virales permettant la recombinaison inter-souches, la complémentation éventuelle de génomes défectifs et l'émergence de souches recombinées dont l'adaptabilité à certains critères peut être fortement accrue. Cela peut notamment permettre d'obtenir une souche hautement adaptée à la culture in vitro ou d'obtenir des souches replicatives à partir de souches défectives.

Le terme "cellules infectées" tel qu'utilisé dans la présente invention fait référence:
i) à des cellules primaires infectées obtenues à partir d'une culture de cellules directement issues d'un prélèvement de tissus ou de fluides biologiques in vivo ou post mortem chez un individu, ainsi qu'aux cellules dérivées obtenues par passages de ces cellules primaires, et
ii) aux cellules secondaires infectées obtenues par coculture de cellules primaires infectées et de cellules permissives, ainsi qu'aux cellules dérivées obtenues par passages de ces cellules secondaires.

Par "cellules primaires", on entend des cellules en culture provenant directement d'un prélèvement de tissus ou de fluides biologiques, et passées en culture sans aucune coculture, ni inoculation de souches virales issues d'autres cellules.

Les cellules prélevées in vivo ou post mortem peuvent être toutes cellules infectées par le virus, par exemple des cellules leptoméningées isolées du liquide céphalorachidien d'un patient (H. Perron et al., Res. Virol., 140, 551-561 (1989)), des cellules myéloïdes trouvées dans le sang, dans le liquide céphalorachidien, dans les tissus ou dans la moelle osseuse, en particulier des macrophages ou monocytes (H. Perron et al., The Lancet, vol. 337, 862-863, 10 avril 1991) et aussi des lymphocytes (S. Haahr et al., The Lancet, vol. 337, 863-864, 6 avril 1991) ou analogues. Un autre candidat pour la préparation d'une culture de cellules primaires est représenté par les cellules de plexus choroïdes humains, qui sont présumées être un site de latence pour un virus associé à la sclérose en plaques.

Le terme "macrophage(s)" fait référence à des cellules dérivant directement de monocytes sanguins, à des cellules résidant dans les tissus (par exemple microgliocytes, cellules de Küpfer) et à des cellules du système réticulo endothélial, notamment les cellules de Langerhans.

Les cellules permissives sont des cellules qui peuvent s'infecter et permettre la replication d'un virus donné avec production de particules virales notamment extra-cellulaires que l'on peut étudier, par exemple pour leur activité transcriptase inverse dans les surnageants.

Le terme "passage" fait réfèrence à une culture cellulaire et correspond à la dissociation des cellules d'un flacon de culture pour les transférer dans un ou plusieurs nouveaux flacons.

Il est bien connu des spécialistes que des modifications spontanées ou induites peuvent survenir dans le caryotype pendant le stockage ou les passages. Ainsi des cellules dérivées d'une lignée cellulaire de référence peuvent ne pas être précisément identiques aux cultures ou cellules d'origine. De même la variabilité génétique des rétrovirus est bien connue, et une souche rétrovirale donnée peut modifier ses caractéristiques par des mutations spontanées ou induites au cours des cultures.

Le matériel biologique viral de l'invention est utilisable directement ou indirectement, à différentes fins, notamment clinique, thérapeutique, diagnostique, ou analytique.

Pour détecter dans tout fluide biologique, prélevé sur le corps humain, la présence d'anticorps dirigés contre un virus de la SEP, il suffit de mettre en contact un échantillon de ce fluide biologique avec, un extrait antigénique d'un matériel biologique viral tel que défini précédemment, ou tout ou partie d'un extrait antigénique immunologiquement analogue audit virus, obtenu par synthèse chimique ou recombinaison génétique, comprenant au moins un épitope d'une souche virale associée à la SEP ; puis on recherche par tout moyen approprié, par exemple par une réaction chromogène ou chromophore, ou radioactive, la présence d'un complexe anticorps-épitope.

L'invention sera mieux comprise à la lecture de la description détaillée qui va suivre, faite en référence aux figures annexées dans lesquelles :
La figure 1, représente l'activité transcriptase inverse de particules concentrées à partir des milieux de culture de la lignée PLI-2, sédimentant sur gradient de saccharose à une densité connue pour les rétrovirus.
Les figures 2 à 5 représentent des particules de type rétroviral observées dans les cellules PLI-2 en microscopie électronique, après transactivation par transfection de plasmides contenant le gène ICPO ou ICP4 du virus Herpes Simplex Type 1. Les échelles sont en µm.

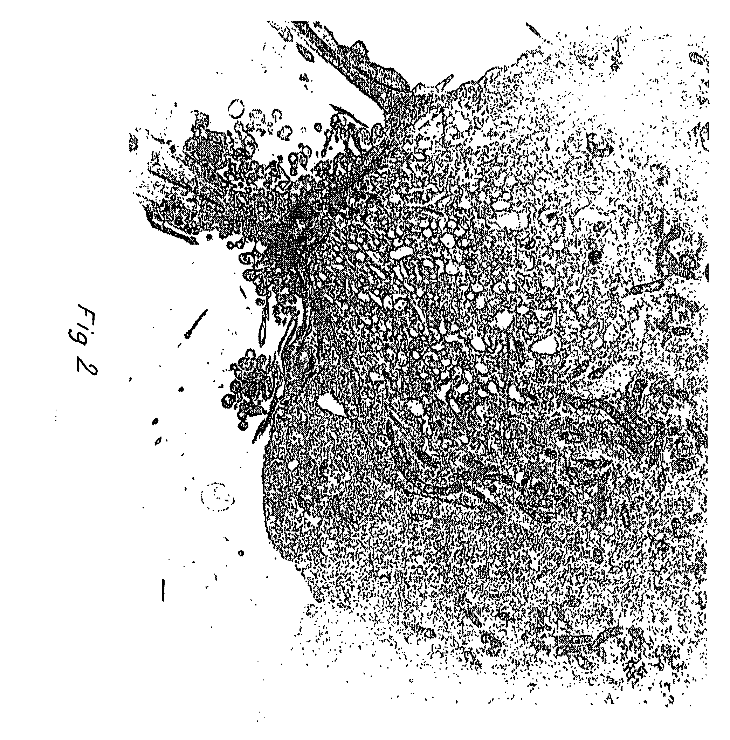

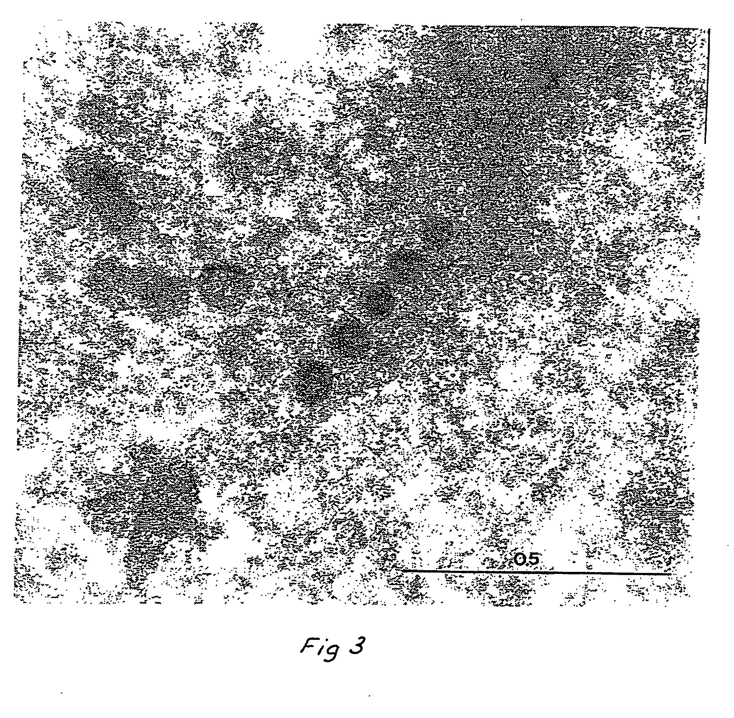

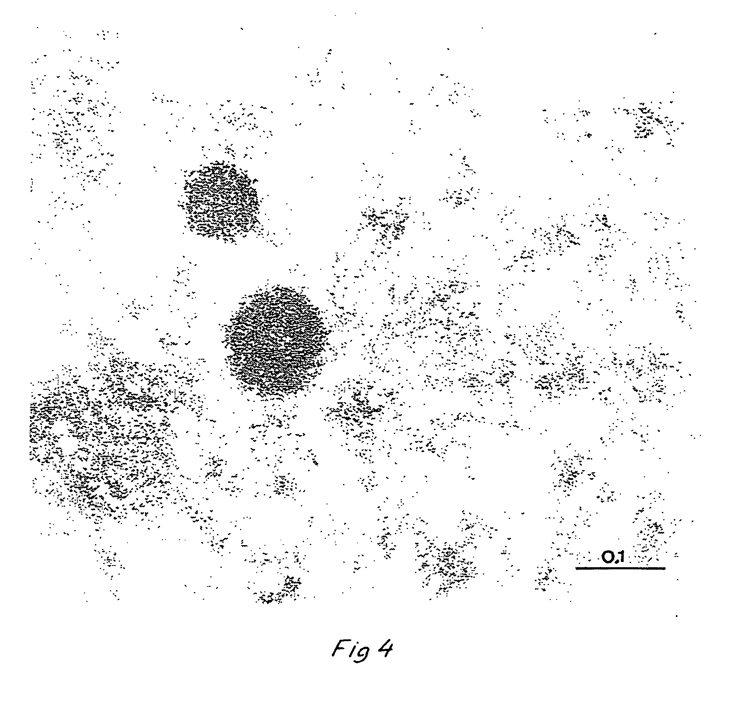

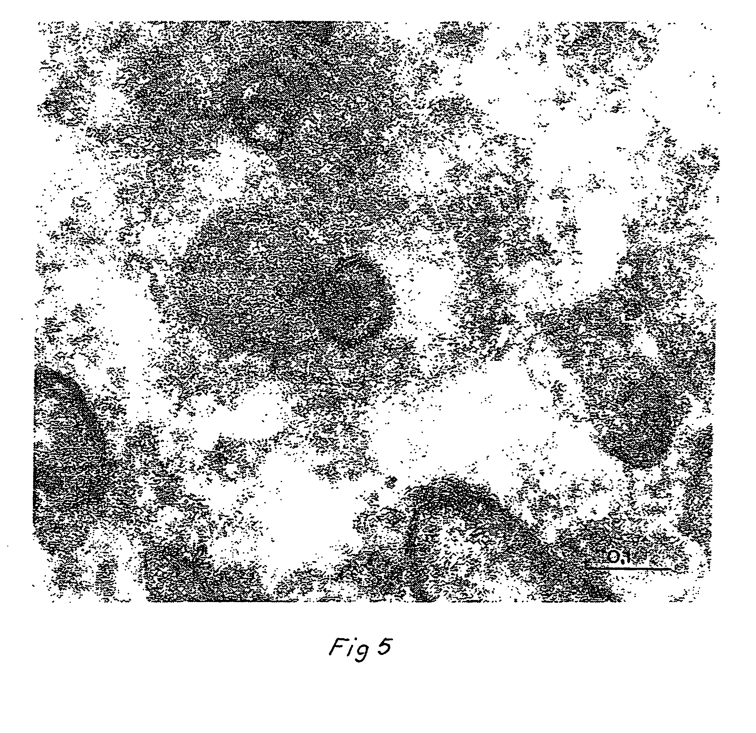

Plus particulièrement, la figure 2 représente une partie de cellule PLI-2 (l'échelle représentée correspond à 1 µm). La figure 3 représente des particules de type rétroviral correspondant à des nucléocapsides intra cytoplasmiques, telles qu'observées en nombre accru après stimulation (transactivation) par le produit du gène ICPO du virus Herpès Simplex Type 1. La figure 4 représente un détail à plus fort grandissement de particules intracytoplasmiques. La figure 5 représente un détail d'une particule d'aspect plus mature, dans les cellules PLI-2 stimulées par transfection avec le gène ICP4 du virus Herpès Simplex Type 1.

### Exemple 1 : Mise en évidence de la production d'interféron beta par les cellules de plexus choroïdes.

Des cellules de plexus choroïdes en culture in vitro infectées par la souche LM7 antérieurement décrite ont été dissociées par une solution de trypsine ou d'EDTA, transférées dans un tube, contenant éventuellement une petite quantité de sérum de veau foetal dans le cas d'une dissociation par la trypsine de cellules résistant à l'action de l'EDTA seul, et culottées par centrifugation à 1600 tpm pendant 5 à 15 minutes. Le culot cellulaire a été resuspendu dans du tampon PBS ("Phosphate Buffer Saline"), et une goutte de suspension a été déposée dans chaque oeillère de quelques lames pour microscope. Après séchage du dépôt, les lames ont été fixées par incubation dans un mélange de méthanol et d'acétone (1 volume/1 volume) à -20°C, pendant 15 minutes.

Alternativement, ces mêmes cellules ont été cultivées sur des lames avec alvéoles de culture (commercialisées par la Société Labtek) et, après rinçage rapide dans du PBS, fixées comme précédemment.

Plusieurs dilutions dans du PBS d'anticorps monoclonal (Boehringer Mannheim, ref. 853 577; dilutions: 1/250, 1/500, 1/1000, 1/2000) ou d'anticorps polyclonal (VIE 3000-ZI LA JUFFARDE 01360 BALAN FRANCE; dilutions: 1/50, 1/100, 1/500, 1/1000) anti-interféron beta humain ont été déposées sur les lames et incubées 3 heures à 37°C. Les lames ont ensuite été rinçées quelques minutes dans deux bains successifs de PBS, puis dans un bain d'eau distillée. Après séchage des lames, des dilutions appropriées d'anticorps marqués au fluorochrome, anti-IgG de souris pour le monoclonal et anti-IgG de chèvre pour le polyclonal, ont été déposées respectivement sur les lames correspondantes qui ont ensuite été incubées pendant 1 heure à 37°C. Les lames ont ensuite été rincées comme précédemment et, après séchage, montées pour l'observation au microscope à fluorescence.

Afin de mieux visualiser les structures cellulaires environnantes, certaines lames ont été incubées environ 1 minute dans une solution de colorant "Bleu Evans", puis rincées et séchées avant montage.

On a ainsi mis en évidence la présence d'une réaction immunologique spécifique révélée par l'émission d'une fluorescence qui confirme la production d'anti-interféron beta par les cellules de plexus choroïdes.

Pour confirmation, des cellules de plexus choroïdes ont été infectées par un virus hautement réplicatif, selon le protocole suivant.

Des cellules de plexus choroïdes non infectées ont été mises en culture dans des flasques de type "Labtek" dans le milieu décrit dans l'exemple 3 ci-après, mais en l'absence d'anticorps anti-interféron β.

Ces cellules ont ensuite été infectées par du virus Herpes simplex type 1 (HSV-1). Elles ont été ainsi été laissées une nuit après un contact d'une heure environ avec un surnageant contenant des virions HSV-1.

Le lendemain, les surnageants ont été prélevés et congelés à-80°C, et les lames constitutives des flasques sur lesquelles les cellules ont poussé ont été dégagées des alvéoles, et fixées au paraformadéhyde puis utilisées, ainsi que des lames de cellules non-infectées, pour une analyse par une technique classique d'immunopéroxydase, avec des anticorps polyclonaux anti-interféron β (exemple : anticorps polyclonal de chèvre anti-interféron β humain commercialisé par VIE-3000, France) et une révélation par un anticorps secondaire marqué à la péroxidase (exemple : anticorps de lapin anti-immunoglobulines de chèvre).

Il a ainsi été mis en évidence un marquage spécifique des cellules de plexus choroïdes infectées par un virus hautement réplicatif tel qu'HSV-1 par les anticorps anti-interféron β; ce qui n'est pas le cas des cellules non-infectées.

Ceci confirme la production d'interféron β par les cellules de plexus choroïde en réponse à une agression virale.

### Exemple 2 : Préparation in vitro d'une culture de cellules primaires infectées par un virus présent chez un patient atteint de SEP.

Les méthodes pour préparer des cultures de cellules primaires à partir de cellules infectées, par exemple des cellules leptoméningées, des monocytes ou des lymphocytes, et les conditions pour leur croissance in vitro sont connues de l'homme du métier (voir références ci-dessus).

### Exemple 3 : Préparation d'une culture de cellules infectées de plexus choroïdes humains.

Des cellules de plexus choroïdes infectées sont obtenues après explantation post mortem de plexus choroïdes humains chez un patient. La pièce anatomique prélevée stérilement est délicatement délacérée avec des pinces et placée dans une solution de trypsine pendant quelques minutes à environ 37°C. Les fragments tissulaires sont recueillis après centrifugation à basse vitesse (500 t/mn) et le surnageant est centrifugé à 16000 t/mn pendant 5 à 15 mn. Le culot de centrifugation est repris dans un milieu RPMI 1640 (commercialisé par Boehringer Mannheim) comprenant : de la penicilline (200.000 U/l), de la streptomycine (200mg/l), de la clindamycine (75 mg/l), de la L-glutamine (6 mM/l), du pyruvate 1%, du sérum, de préférence du sérum de veau foetal 20 à 30% décomplémenté par incubation à 56°C pendant 30 minutes, des acides aminés non essentiels 1% (Boehringer Mannheim MEM, A.A.N.E. 100X ref: 210293) et de l'anticorps anti-interféron beta humain, polyclonal tel que commercialisé par VIE 3000, ou monoclonal ayant une activité neutralisante vis-à-vis de l'interféron beta (10U/ml). Avantageusement, le milieu de culture comprend de plus un facteur de croissance tel que le facteur de croissance de cellules endothéliales (ECGF) associé à de l'héparine (BOEHRINGER ref. 1/79/87 : ECGF environ 1 à 20 ng/ml comprenant de l'héparine 50-150 µg/ml).

Les cellules sont maintenues dans leur milieu de culture et laissées dans une étuve à CO₂ jusqu'à ce que la prolifération cellulaire produise une couche confluente, c'est à dire un tapis de cellules adhérantes. A ce stade, les cellules sont dissociées avec une solution d'EDTA (Versène). Les cultures de cellules sont ensuites dédoublées régulièrement aussi longtemps que le potentiel mitotique le permet. Les milieux de culture sont changés au moins deux fois par semaine et toujours le lendemain d'un nouveau passage, c'est-à-dire à chaque nouvel ensemencement d'un flacon avec des cellules dissociées en suspension.

On observe une extinction progressive du pouvoir mitotique des cultures infectées après une quinzaine de passages environ, ce qui est conforme aux observations faites en l'absence d'anticorps anti-interféron beta. Mais, fait jamais observé dans le procédé de culture de cellules de plexus choroïdes infectées in vitro dans un milieu de culture dépourvu d'anticorps anti-interféron beta, quelques foyers de prolifération clonale sont progressivement apparus après le dernier passage des cellules de plexus choroïdes infectées prélevées chez un patient atteint de SEP. Trois de ces foyers ont pu être repiqués et l'un d'entre eux s'est établi en une lignée à fort potentiel de pousse in vitro. Cette lignée a été dénommée PLI-2 par la Demanderesse.

Les surnageants de culture de cette lignée établie de cellules de plexus choroïdes infectées, d'un volume minimal de 15 ml, ont été collectés, précentrifugés à 10.000 tpm pendant 30 minutes pour éliminer les débris cellulaires, puis ultracentrifugés à 35.000 tpm (100000 g) pendant 2 heures pour sédimenter les particules rétrovirales. Les culots sont repris (volume final concentré environ 1.000 fois dans du tampon, Tris-HCl 0,05M pH 9,5) et conservés à -80°C pour un dosage ultérieur de l'activité transcriptase inverse et la préparation de virus concentré comme décrit ci-après.

### Exemple 4 : Dosage de l'activité transcriptase inverse pour le suivi de la production de particules virales de type LM7 dans le surnageant des cellules de PLI-2.

Toutes les étapes sont effectuées avec du matériel et des solutions stériles, afin d'éviter toute interférence avec des nucléases ou protéases bactériennes, notamment pendant les phases d'incubation à 37°C.

Les culots contenant les particules virales concentrées dans du Tris HCl 0,01M pH8 sont décongelés et homogénéisés. 20 µl sont prélevés et ajoutés dans un mélange réactionnel contenant : 5 µl de (Tris 0.5M + DTT 0,04M) pH 8.2, 5 µl de NaCl 0.1M, 5 µl de MgCl₂ 0.3M, 23 µl d'H₂O bidistillée, 10 µl de NP₄O 2%, 2 µl polyCm-oligodG12-18 (10 U D.O./ml ; Pharmacia) et 5 µl 3H, 3H-Guanosine-Tri-Phosphate (1 mCi/ml ; NEN). Les tubes en verre contenant les mélanges sont incubés à 37°C pendant 75 minutes. La réaction est arrêtée en ajoutant 75 µl d'une solution à +4°C contenant : 12,5 % H₂O saturée avec du phosphate de sodium, 12,5 % H₂O saturée avec du pyrophosphate de sodium, et 20 % d'acide Tri-Chloro-Acetique (TCA). Après 30 min. à 1 heure à 4°C, les tubes sont remplis avec une solution de TCA 5 %, vidés et rincés 5 fois avec la solution de TCA à 5 %, sur une membrane d'acétate de cellulose (Sartorius ref. 11106 25 N ; diamètre de pore : 0,45 µ ; diamètre de la membrane : 25mm) à travers laquelle les échantillons sont filtrés dans un collecteur de fractions 1125 (Millipore ; ref. XX2702550). Avant d'être enlevées, les membranes sont rincées encore une fois avec 20 ml de TCA à 5 %. Les membranes sont alors placées dans des fioles que l'on remplit de liquide scintillant (Ready-Safe, Beckman) et l'activité est mesurée dans un compteur beta, en cpm (coups par minute) et dpm (désintégrations par minute).

Chaque échantillon est testé en triple et la moyenne des valeurs utilisée comme résultat. Si la différence entre cette moyenne et l'une des mesures excède deux fois l'écart type mesuré sur des valeurs de référence, l'échantillon correspondant est testé à nouveau.

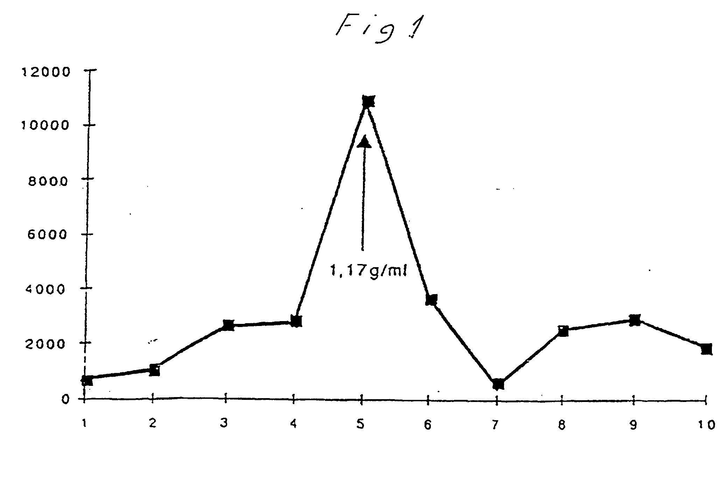

Pour vérifier que l'activité transcriptase inverse est bien associée à des particules de type rétroviral, les culots de virion concentrés par ultracentrifugation des surnageants de culture sur coussin de glycérol (solution PBS + 30 % de glycérol) sont placés sur des gradients de saccharose (15 à 50 % poids/poids) et ultracentrifugés à + 4°C pendant 6 à 16 H à 35.000 tpm (100.000 g) dans un rotor à godets. 10 fractions sont recueillies et 20 µl sont prélevés dans chaque fraction pour y doser l'activité transcriptase inverse comme décrit ci-dessus. Le pic spécifique d'activité est retrouvé dans la fraction de densité 1,17 g/ml environ (dosage réfractométrique), ce qui correspond à une densité de sédimentation à l'équilibre connue pour les particules rétrovirales (1,16 à 1,18 g/ml) (Figure 1).

### Exemple 5 : Transfection de cellules de la lignée PLI-2 par des gènes "immédiat-précoces" du virus de l'Herpès Simplex de Type 1.

Ainsi que cela a été montré (Perron et al. Res. Virol. 1989), le virus herpes simplex type 1 (HSV-1) stimule la production de virus LM7 dans les cellules leptoméningées. Par ailleurs, HSV-1 s'avère transactiver l'expression du rétrovirus HIV par le produit d'un de ses gènes "immédiats-précoces", ICPO ou IE1 (Chapman J.C., Harris J.D., Collins M.K.L. & Latchman, D.S. 1991, A recombinant HIV provirus is synergistically activated by the HIV Tat protein and the HSV IE1 protein but not by the HSV IE3 protein. AIDS 5, 945-950 et Mosca, J .D. Bednarik, D.P. Raj, N.B. Rosen, C.A. Sodroski, J.G. Haseltine, W.A. & Pitha, P.M. 1987. Activation of human immunodeficiency virus by herpesvirus infection : identification of a region within the long terminal repeat that responds to a trans-acting factor encoded by herpes simplex virus 1. Proceedings of the National Academy of Sciences 84, 7408-7412).

Sur la base de ces constatations, les inventeurs ont alors envisagé d'utiliser les gènes "immédiat-précoces" du virus HSV-1 pour accroître l'expression virale par la lignée cellulaire PLI-2.Différents plasmides contenant chacun un gène immédiat-précoce du virus HSV-1 ont donc été testés par transfection dans les cellules de plexus choroïdes infectées. Il a ainsi été mis en évidence, par microscopie électronique et dosage de l'activité transcriptase inverse, que les gènes ICPO (cloné dans le plasmide pJR3) et ICP4 (cloné dans le plasmide XhoC) transactivaient fortement l'expression d'un virus semblable au LM7 dans les cellules de la lignée PLI-2 (figures 2 à 5). Des particules virales d'environ 100 nm sont retrouvées dans les culots d'ultracentrifugation de surnageants de ces cultures exprimant une activité transcriptase inverse accrue.

Le protocole expérimental de la transfection est décrit ci-dessous.

Le plasmide pJR3 contient un fragment de restriction Pst I-Sacl d'HSV-1 (nucléotides 18663 à 25066) codant pour ICPO, cloné dans un vecteur pUC9. Le plasmide XhoC est un fragment de restriction Xhol d'HSV-1 (nucleotides 23028 à 33520) codant pour ICP4, cloné dans un vecteur pAT153.

La transfection est réalisée avec le Transfectam (marque enregistrée, SEPRACOR, Villeneuve la Garenne, France). Le principe de cette transfection consiste en une interaction spécifique entre une lipopolyamine cationique et l'ADN du plasmide.

Les plasmides sont dilués dans une solution stérile de NaCl 0,3 M dans la proportion de 2 µg/250 µl et de 5 à 7 µg/10⁶ cellules. Cette solution est mélangée immédiatement avant la transfection avec le même volume d'eau bi-distillée contenant 5 µl de solution stock de Transfectam par µg de plasmide. Ce mélange Transfectam-plasmide est alors versé sur les cultures préalablement lavées avec du RPMI sans sérum (2 x 15 min.), et mélangé à un volume minimum de milieu selon l'invention, mais sans sérum de veau foetal, laissé dans les flacons de manière à couvrir la surface de la monocouche de cellules adhérentes. Les flacons de culture sont alors incubés 6 h dans une étuve avec 5 % de CO2, à 37°C. Après 6 heures, le mélange Transfectam-plasmide est prélevé et remplacé par du milieu de culture selon l'invention.

Les cellules sont réincubées dans l'étuve et les surnageants prélevés environ un jour après les 6 h de contact avec le mélange de transfection, puis chaque jour pendant une semaine. Les surnageants, dont celui qui contient le Transfectam avec le plasmide, sont conservés à -80°C, ou concentrés comme décrit dans l'exemple 6 ci-après, et utilisés par la suite comme source enrichie de virions.

L'expression des gènes ICPO ou ICP4 dans les cellules transfectées est vérifiée par immunofluorescence indirecte avec des anticorps polyclonaux de lapin ou monoclonaux de souris contre les protéines produites par ces gènes.

### Exemple 6 : Préparation de virus purifié concentré à partir des surnageants de culture de la lignée PLI-2.

Pour la préparation de virus concentré et purifié, un volume de surnageant obtenu selon l'exemple 3 ou 5 (entre 5 et 10 litres) est décongelé et concentré par ultrafiltration tangentielle (système Minitan,Millipore) sur une série de membranes ayant un seuil de coupure à 300000 daltons. Le concentrat est ensuite centrifugé selon l'exemple 3 mais avec reprise du culot dans du tampon PBS. Les culots ainsi recueillis sont alors regroupés et déposés sur un gradient de saccharose dans un tampon PBS stérile (15 à 50% poids/poids) et ultacentrifugé à 35 000 tpm (100 000 g) pendant 6 heures à +4°C dans un rotor à godets. 10 fractions sont recueillies et 20 µl sont prélevés dans chaque fraction pour y doser l'activité transcripase inverse selon la technique précédemment décrite. Les fractions contenant le pic spécifique d'activité correspondant à une densité de sédimentation à l'équilibre comprise entre 1,16 et 1,18 g/ml connue pour les particules rétrovirales sont ensuite diluées dans du tampon PBS stérile et ultracentrifugées une heure à 35000 tpm (100000 g) pour sédimenter les particules virales. Le culot de virions purifiés ainsi obtenu est alors repris dans un petit volume d'un tampon adéquat pour l'utilisation ultérieure qui en sera faite (ex: Tampon Guanidium Thiocyanate pour l'extraction des ARN; PBS stérile pour le stockage à -80°C).

Alternativement, de grandes quantités de flacons de cellules PLI-2 peuvent être mis en culture et les surnageants dont le volume total dépasse 2 litres à chaque changement de milieu, sont pré-centrifugés à 10000 tpm, puis directement concentrés avec le système Minitan (marque enregistrée, Millipore), sans congélation intermédiaire.

### Exemple 7 : Coculture d'une lignée cellulaire infectée par un virus présent chez un individu atteint de SEP et de cellules non infectées permissives au virus.

Des cellules d'une culture primaire infectées, telles que décrites dans l'exemple 2, par un virus présent chez un individu atteint de SEP, par exemple le virus LM7, sont prélevées de leur flacon de culture, puis reprises dans un milieu de culture approprié à la coculture, c'est-à-dire le milieu de culture des cellules de plexus choroïdes infectées décrit à l'exemple 3, avec un anticorps anti-interféron beta humain.

Parallèlement, des cellules de plexus choroïdes non infectées obtenues après explantation post mortem de plexus choroïdes humains sont cultivées dans les conditions de l'exemple 3, c'est-à-dire avec un anticorps anti-interféron beta humain. Puis, ces cellules sont dissociées de leur flacon de culture dans une solution de trypsine-EDTA. Les cellules sont ensuite centrifugées et resuspendues dans leur milieu de culture, et sont rajoutées au flacon de la culture cellulaire infectée. Le flacon est placé dans une étuve à CO₂ et on laisse les cellules de plexus choroïdes adhérer et proliférer au fond du flacon contenant déjà les cellules infectées pendant 24 H. Le milieu est changé après 24 H et le mélange de cellules est laissé dans l'étuve à CO₂, jusqu'à ce que la prolifération de cellules permissives de plexus choroïde produise une couche confluente, c'est-à-dire un tapis de cellules adhérentes. A ce stade, les cellules sont maintenues encore 5 à 7 jours pour assurer le transfert du virus des cellules infectées aux cellules de plexus choroïdes. La culture cellulaire est ensuite dédoublée et passée dans deux nouveaux flacons qui sont réensemencés avec les cellules de plexus choroïdes dissociées en suspension, et soumises aux mêmes conditions que décrites ci-dessus pour l'adhésion et la prolifération des cellules, le transfert, l'expression et la réplication du virus. Les cultures de cellules sont ensuite dédoublées régulièrement et passées aussi longtemps que le potentiel mitotique des cellules permissives le permet. Ces cellules, qui hébergent et produisent un virus du type LM7 peuvent à leur tour être utilisées pour infecter de nouvelles cellules par coculture comme décrit précédemment, et ainsi maintenir la souche virale en culture.

Les milieux de culture sont toujours changés au moins deux fois par semaine et toujours le lendemain d'un nouveau passage, c'est-à-dire à chaque nouvel ensemencement d'un flacon avec des cellules dissociées en suspension.

Préalablement à la coculture, les cellules hébergeant la souche virale peuvent éventuellement être irradiées de manière à éviter leur prolifération ultérieure au sein d'une culture nouvellement infectée. L'irradiation peut par exemple être réalisée avec une dose totale de 6.000 rad de rayons X.

On observe une extinction du pouvoir mitotique après une quinzaine de passages environ, ce qui est conforme aux observations faites précédemment en l'absence d'anticorps anti-interféron beta.

Cependant, comme mis en évidence avec la culture de cellules de plexus choroïdes infectées de l'exemple 3, quelques foyers de prolifération clonale sont apparus dans plusieurs flacons en culture, après le dernier passage. Ce même phénomène a conduit à l'apparition de cellules à fort potentiel mitotique, qui ont supplanté les cellules pré-sénescentes dans une sous-culture de la souche LM7 (dénommée LM7 PG).

Les surnageants de culture de cette lignée établie ont été collectés et congelés à -80°C selon le protocole décrit dans l'exemple 3.

Le dosage de l'activité transcriptase inverse pour le suivi des particules virales de type LM7 dans le surnageant de cellules LM7 PG a été effectué conformément au protocole expérimental décrit dans l'exemple 4, et a permis de mettre en évidence un pic d'activité spécifique dans la fraction de densité 1,17 g/ml environ, ce qui correspond à une densité connue pour les particules rétrovirales (figure 1).

Les cellules de la lignée cellulaire LM7 PG ont ensuite été transfectées, selon un protocole identique à celui décrit dans l'exemple 5, ce qui a permis de montrer que les gènes ICPO (plasmide pJR3) et ICP4 (plasmide XhOC) transactivent fortement l'expression virale dans les cellules de la lignée LM7 PG. Les particules virales produites présentent les caractéristiques des rétrovirus.

La préparation de virus purifié concentré est effectuée à partir des surnageants obtenus soit directement après coculture, soit par transfection des cellules de la lignée LM7 PG, avec les gènes ICPO et/ou ICP4 du virus HSV-1.

## Revendications

1. Souche virale humaine, à l'état isolé dénommée POL-2, déposée à l'ECACC le 22 juillet 1992, sous le N° V92072202 selon les dispositions du Traité de Budapest et hébergée par la lignée cellulaire dénommée PLI-2, déposée à l'ECACC le 22 juillet 1992 sous le N° 92072201 selon les dispositions du Traité de Budapest

## Patentansprüche

1. Isolierter menschlicher Virus-Stamm, POL-2 benannt, der am 22. Juli 1992 unter der Nummer V92072202 gemäß den Vorschriften des Budapester Vertrages bei der ECACC hinterlegt wurde und enthaltet in der PLI-2 benannten Zellinie, die am 22. Juli 1992 unter der Nummer 92072201 gemäß den Vorschriften des Budapester Vertrages bei der ECACC hinterlegt wurde

## Claims

1. Human viral strain, in the isolated state, called POL-2, deposited at the ECACC on 22 July 1992 under No. V92072202 in accordance with the provisions of the Treaty of Budapest, and harboured by the cell line called PLI-2, deposited at the ECACC on 22 July 1992 under No. 92072201 in accordance with the provisions of the Treaty of Budapest.
